(19) Europäisches Patentamt

European Patent Office

Office européen des brevets

(11) **EP 4 651 145 A1**

(12) **EUROPEAN PATENT APPLICATION**

(43) Date of publication:
**19.11.2025 Bulletin 2025/47**

(21) Application number: **25174485.0**

(22) Date of filing: **06.05.2025**

(51) International Patent Classification (IPC):
**G16H 20/17** *(2018.01)*    **G16H 50/50** *(2018.01)*

(52) Cooperative Patent Classification (CPC):
**G16H 20/17; G16H 50/50**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **16.05.2024  FR 2405053**

(71) Applicant: **Diabeloop**
**38000 Grenoble (FR)**

(72) Inventors:
- **ROMERO-UGALDE, Hector**
  **38420 Le Versoud (FR)**
- **GAUTHIER, Pierre**
  **38120 Saint Egreve (FR)**
- **HUNEKER, Erik**
  **38120 Saint Egreve (FR)**
- **LE ROUX-MALLOUF, Thibault**
  **38100 Grenoble (FR)**

(74) Representative: **Lannel, Pierre Olivier**
**EINNOVS**
**334, Chemin de la Chapuise**
**69560 Saint-Cyr-sur-le-Rhône (FR)**

(54) **CLOSED-LOOP BLOOD GLUCOSE CONTROL SYSTEMS AND METHODS**

(57) A control device (30) for determining a recommendation value of a control parameter of an insulin infusion device (20).

[Fig. 1]

## Description

### Field of the invention

The present invention relates to the field of closed-loop blood glucose control systems for the controlled delivery of insulin to a patient. Such systems are also known as artificial pancreas.

### Background of the invention

An artificial pancreas is a system that automatically regulates the insulin intake of a diabetic patient, or user, based on its measured blood glucose history, meal history, and insulin history.

In particular, the present invention relates to Insulin Sensitivity Factor (ISF), which may be utilized to determine the appropriate insulin dose based on the sensitivity of the patient's body to insulin allowing for a more precise adjustment of insulin dosing to maintain optimal glycemic control.

It would be desirable to enhance the performance of Insulin Sensitivity Factor-based systems by improving the accuracy and reliability of physiological models for example. More accurate predictions of insulin sensitivity would enable better estimation of insulin requirements, thereby reducing the risks of both hyperglycemia and hypoglycemia. This improvement in prediction accuracy is critical for optimizing insulin therapy and ensuring better overall management of diabetes.

US20090054753A1 discloses insulin sensitivity as a general measure of the body's response to insulin dosing. This factor can change as the patient's physiological status changes and can be useful in determining the patient's response to therapy. The patient's insulin sensitivity can be determined in various ways, for example by input from a care provider, by inference from other conditions, or by determination from previous insulin dosing and glucose measurement information. Insulin sensitivity can be utilized as a parameter to determine the next sampling time.

The present invention aims at improving this situation.

The invention thus aims to answer at least partially the above presented technical problems.

Brief summary of the invention

Thus, the invention relates to a control device for determining a recommendation value of a control parameter of an insulin infusion device, the control device comprising:

- a retrieving unit, the retrieving unit being configured to retrieve user data, each data of the user data having a timestamp and the user data being related to a unique user, the user data comprising at least:

    - an amount of insulin infused to the unique user;
    - an amount of carbohydrates ingested by the unique user;
    - a plurality of physiological values of the unique user, the plurality of physiological values of the unique user comprising at least measured blood glucose levels;

- a recommendation unit, the recommendation unit being configured to determine the recommendation value at least on the basis of an Insulin Sensitivity Factor (ISF);

wherein, the ISF is a function of at least a measured blood glucose level of the plurality of physiological values.

Using an ISF being a function of at least a measured blood glucose level allows the recommendation unit to more accurately determine a recommendation value fitting the unique user's needs as the ISF can vary from one unique user to another and from one measured blood glucose level to another.

According to the present invention, the ISF is representative of the effect of a determined dose of insulin on a blood glucose level of the unique user.

According to the present invention, the ISF is representative of the effect of a unit of insulin on the measured blood glucose level of the unique user. A unit of insulin corresponds to the "biological equivalent" of 34.7 $\mu$g pure crystalline insulin.

According to the present invention, the ISF being a function of at least a measured blood glucose level of the plurality of physiological values imply that the ISF changes depending on the measured blood glucose level and therefore changes over time if the measured blood glucose level is not constant over time.

According to an embodiment, the control device also comprises an insulin delivery unit such as a subcutaneous insulin delivery device configured to deliver exogenous insulin in a subcutaneous tissue of the patient in response to an insulin delivery control signal, in particular continuously infused insulin such as basal insulin and/or bolus insulin.

According to an embodiment, the subcutaneous insulin delivery device is an insulin pump.

According to an embodiment, the recommendation value is a recommendation value corresponding to an amount of insulin to be injected at a future time step.

According to an embodiment, the future time step is no more than a few seconds after the nearest timestamp of the physiological values of the unique user. This delay of no more than a few seconds corresponds to the computational time required to determine the recommendation value. The recommendation value can be of any type such as a bolus recommendation or a basal recommendation for example.

According to the present invention, the terms injected or injection must be understood as a virtual injection in case the training of the reinforcement learning algorithm is made using a simulation wherein the unique user is a virtual user.

According to an embodiment, the control device comprises a converter unit, the converter unit being configured to convert the recommended value of insulin into a control parameter of the fluid infusion device. According to an embodiment, the control parameter takes the form of a bolus and/or a basal. Such a configuration allows the infusion device to infuse the recommendation value to the unique user.

According to the present invention, a measured blood glucose level is a blood glucose level measured on the unique user. The blood glucose level can be measured by any means such as a Continuous Glucose Monitor (CGM) or a Blood Glucose Monitor (BGM) for example.

According to the present invention an amount of carbohydrates ingested by the unique user corresponds to an amount of sugar ingested in a meal for example.

According to an embodiment, the ISF is a function of measured blood glucose levels having a timestamp not older than one to three hours prior to the present time and preferably two hours prior to the present time. Such a configuration allows the recommendation unit to more accurately determine the recommendation value as measured blood glucose levels having older timestamps are not as representative of the unique user state as the measured blood glucose levels having younger timestamps.

According to an embodiment, the ISF is a decreasing function of at least a measured blood glucose level of the plurality of physiological values.

Such a configuration allows the control device to accurately determine a recommendation value and therefore helps manage the blood glucose levels of the unique user. Indeed, an ISF being a decreasing function of at least a measured blood glucose level of the plurality of physiological values allows the recommendation unit to accurately determine a recommendation value.

According to an embodiment, the ISF is a decreasing function of at least a measured blood glucose level of the plurality of physiological values wherein the considered at least a measured blood glucose level of the plurality of physiological values is greater than 100 mg/dl.

According to an embodiment, the ISF is a function of at least a measured blood glucose level of the plurality of physiological values as follows:

$$ISF = m \times Gly + b$$

Gly being the considered at least a measured blood glucose level of the plurality of physiological values such as Gly being the closest, in time, measured blood glucose level for example;
m being a factor; and
b being a constant.

Such a configuration allows the control device to accurately determine a recommendation value and therefore helps manage the blood glucose levels of the unique user. Indeed, an ISF being a decreasing function of at least a measured blood glucose level of the plurality of physiological values allows the recommendation unit to accurately determine a recommendation value.

According to an embodiment, m varies depending on Gly such as m in [1; 1.5] for Gly below 100 mg/dl, m in [-0.8; -0.6] for Gly in [100; 160[ mg/dl and m in [-0.2; 0] for Gly above 160 mg/dl. Such a configuration allows the ISF to be a closer representation of a physiological state of the unique user. According to an embodiment, m varies depending on Gly by smoothly transitioning from 1 to -0.8 according to the previously described evolutions of m.

According to an embodiment, b varies depending on Gly such as b in [-50; -30] for Gly below 100 mg/dl, b in [150; 170] for Gly in [100; 160[ mg/dl and b in [50; 70] for Gly above 160 mg/dl. Such a configuration allows the ISF to be a closer representation of a physiological state of the unique user. According to an embodiment, b varies depending on Gly by smoothly transitioning from -50 to 170 according to the previously described evolutions of b. According to another embodiment, ISF is a piecewise affine function with an undefined number of pieces.

According to an embodiment, the ISF is a piecewise affine function of Gly with m=m1 and b=b1 for Gly below 100 mg/dl, m=m2 and b=b2 for Gly in [100; 160 [ mg/ dl and m=m3 and b=b3 for Gly above 160 mg/dl. Such a configuration allows the ISF to be a closer representation of a physiological state of the unique user. According to another embodiment, ISF is a piecewise affine function with an undefined number of pieces. The functions in the piecewise affine function are non linear exponential functions for instance $ISF(Gly) = a \times e^{\wedge}((c \times Gly))$.

The control device 30 is configured to modify m and b as well as m1, m2, m3, b1, b2 and b3 over time. Such a configuration allows the control device 30 to more precisely adapt to the specificities of the unique user. m and b as well as m1, m2, m3, b1, b2 and b3 can be modified over time using any known method such as the autolearning method described in details above applied to the ISF for example.

According to an embodiment, functions in the piecewise affine function are logarithmic functions $a \times \log(Gly) + b$.

Note that previously described examples of piecewise affine functions are linear and nonlinear functions. The piecewise affine functions could be a mix of linear and nonlinear functions such as linear for Gly < 100 mg/dL and nonlinear for Gly >=100 mg/dL for example.

According to an embodiment, the recommendation unit is configured to determine the recommendation value at least on the basis of a predicted glucose level determined by computing a physiological model of glucose-insulin system representative of the unique user using at least a portion of the user data and the ISF.

Such a configuration allows one to accurately determine a recommendation value and therefore helps manage the blood glucose levels of the unique user. Indeed, using an ISF allows one to adapt the recommendation value specifically to the unique user. Furthermore, an ISF being a function of at least a measured blood glucose level of the plurality of physiological values allows the physiological model to closely reproduce the physiology of the unique user and therefore allows the recommendation unit to accurately determine a recommendation value.

Such a configuration also allows one to more accurately test or fine tune an existing algorithm using a simulator for example. Indeed, an improved physiological model more closely reproduces the physiology of the unique user and therefore allows one to more accurately evaluate the existing algorithm.

According to an embodiment, the physiological model can be of any type such as the Hovorka model, the Bergman minimal model or the Dalla Man Model for example. According to a preferred embodiment, the physiological model is the Hovorka model or a model derived from the Hovorka model.

According to an embodiment, the recommendation unit is configured to determine the recommendation value at least on the basis of a Product Integral Derivative (PID) approach and an ISF. The PID is based on at least a measured or predicted blood glucose level and at least a blood glucose level target, the ISF is then used as a weighting factor.

According to an embodiment, the recommendation unit is configured to determine the recommendation value at least on the basis of a Neural Network and an ISF. The Neural Network uses as an input at least an amount of insulin infused to the unique user, at least an amount of carbohydrates ingested by the unique user and at least a physiological value of the unique user and outputs a raw recommendation value, the ISF is then used as a weighting factor. The Neural Network could be of any type and trained as follows:

- Collect Data;
- Preprocess Data;
- Design a neural network architecture such as a regression model for example;
- Define the input features;
- Define the output features;
- Train the neural network using the training preprocessed data;
- Validate the model's performance; and
- Evaluate the model's performance using metrics such as Mean Absolute Error (MAE) or Mean Squared Error (MSE) for example.

According to an embodiment, the recommendation unit is configured to determine the recommendation value at least on the basis of a predicted glucose level determined by computing a physiological model of glucose-insulin system representative of the unique user using an action speed of insulin wherein the action speed of insulin is an increasing function of at least a measured blood glucose level of the plurality of physiological values.

Such a configuration allows one to obtain a better Insulin On Board (IOB) value and therefore allows the recommendation unit to more accurately determine a recommendation value.

According to an embodiment, the at least a measured blood glucose level of the plurality of physiological values corresponds to the last measured blood glucose level of the plurality of physiological values or the few last measured blood glucose levels of the plurality of physiological values, or an average of the few last measured blood glucose levels of the plurality of physiological values.

According to an embodiment, the ISF is not a function of a Total Daily Dose nor, *a fortiori,* a mean of several past Total Daily Doses. The Total Daily Dose being the total amount of insulin infused to the unique user over the course of a day. Indeed, the ISF is not a constant of glycemia level.

According to an embodiment, the ISF is a decreasing function of at least a measured blood glucose level of the plurality of physiological values and depends on at least one past ISF.

Such a configuration allows one to accurately determine a recommendation value and therefore helps manage the blood glucose levels of the unique user while reducing the risk for the unique user. Indeed, an ISF depending on at least one past ISF allows one to avoid abrupt changes in the determination of the recommendation value.

According to the present invention, a past ISF is an ISF previously calculated. Such a past ISF can be, for example, calculated the same way as the ISF or can be a past ISF initialized arbitrarily.

According to an embodiment, the ISF is a decreasing function of at least a measured blood glucose level of the plurality of physiological values and depends on an average of several past ISF.

Such a configuration allows one to further smooth the changes in the determination of the recommendation value and therefore reduce the risk for the unique user.

According to an embodiment, the ISF depends on an autolearned glycemia factor such as ISF = avgISF * Gly * glyF

wherein:

avgISF being an average of past ISF; and
glyF being a glycemia factor.

According to an embodiment, avgISF is the average of all the computed ISF over the course of a week.

According to an embodiment, the ISF is a decreasing function of at least a measured blood glucose level of the plurality of physiological values and depends on an autolearned ISF.

According to an embodiment, the ISF depends on an autolearned glycemia factor such as ISF = ALISF * Gly * glyF
wherein:
ALISF being the autolearned ISF;

Such a configuration allows one to accurately determine a recommendation value and therefore helps manage the blood glucose levels of the unique user by adapting to the unique user over time. Such a configuration is especially useful when the variability of the unique user's ISF depending on the measured blood glucose level is high.

Such a configuration allows one to accurately determine a recommendation value and therefore helps manage the blood glucose levels of the unique user by adapting to the unique user over time. Indeed, the ISF of the unique user can vary depending on several factors such as illness unrelated to diabetes for example.

According to the present invention, the term "autolearned ISF" relates to an ISF determined using an autolearning method. The autolearning method could be of any type such as a process through which a system, typically a computer program or an artificial intelligence, autonomously acquires knowledge, skills, or behaviors without explicit programming or direct human intervention. Autolearning systems often rely on algorithms, data, and feedback mechanisms to improve their performance over time, adapting to changing conditions or tasks through self-adjustment and refinement.

According to an embodiment, the autolearned ISF is determined by creating a plurality of time segments, each time segment having an ISF, in order to group user data at least based on their timestamp; creating a segment correction value for each time segment, the segment correction values being based at least on one past ISF and on at least one physiological value of the plurality of physiological values during a time segment of the plurality of time segments; creating the autolearned ISF for a future determined period of time, wherein the autolearned ISF is based at least on a segment correction value of the segment correction values and the ISF of the determined period of time. Using segments allows one to accurately determine the autolearned ISF as each segment correction values can be created differently, time segment by time segment or depending on a specific interest of a time segment for example.

According to an embodiment, the ISF depends on an autolearned glycemia factor.

According to an embodiment, the ISF depends on an autolearned glycemia factor such as ISF = pISF * Gly * ALglyF
wherein:

pISF being a past ISF;
avgISF being an average of past ISF such as the closest, in time, past five ISF for example;
ALglyF being the autolearned glycemia factor.

Such a configuration allows one to accurately determine a recommendation value and therefore helps manage the blood glucose levels of the unique user by adapting to the unique user over time. Such a configuration is especially useful when the variability of the unique user's ISF depending on the measured blood glucose level is high.

According to an embodiment, the pISF can be replaced by avgISF. Indeed, ISF = avgISF * Gly * ALglyF allows one to further smooth the changes in the determination of the recommendation value and therefore reduce the risk for the unique user.

According to an embodiment, ALglyF is a combination of a factor and a constant term.

According to the present invention, the term "autolearned glycemia factor" relates to a glycemia factor determined using an autolearning method. The autolearning method could be of any type such as a process through which a system, typically a computer program or an artificial intelligence, autonomously acquires knowledge, skills, or behaviors without explicit programming or direct human intervention. Autolearning systems often rely on algorithms, data, and feedback mechanisms to improve their performance over time, adapting to changing conditions or tasks through self-adjustment and refinement.

According to an embodiment, the autolearned glycemia factor is determined by creating a plurality of time segments, each time segment having a glycemia factor, in order to group user data at least based on their timestamp; creating a segment correction value for each time segment, the segment correction values is based at least one past glycemia factor and at least one physiological value of the plurality of physiological values during a time segment of the plurality of time segments; creating the autolearned glycemia factor for a future determined period of time, wherein the autolearned glycemia factor is based at least on a segment correction value of the segment correction values and the glycemia factor of the determined period of time. Using segments allows one to accurately determine autolearned glycemia factor as each segment correction values can be created differently, time segment by time segment or depending on a specific interest of a time segment for example.

According to an embodiment, the ISF is a decreasing function of at least a measured blood glucose level of the plurality of physiological values and depends on an autolearned ISF and an autolearned glycemia factor.

According to an embodiment, the ISF depends on an autolearned ISF and an autolearned glycemia factor such as ISF = ALISF * Gly * ALglyF.

Such a configuration allows one to accurately determine a recommendation value and therefore helps manage the blood glucose levels of the unique user by adapting to the unique user over time. Indeed, both the ISF and the glyF of the unique user can vary depending on several factors such as illness unrelated to diabetes for example.

According to an embodiment, the recommendation unit is configured to determine the recommendation value at least on the basis of the ISF and a Correction Factor.

According to an embodiment, the Correction Factor is a factor applied to the ISF and allows the recommendation unit to take into account particular situations of the unique user such as illness unrelated to diabetes for example.

According to an embodiment, the recommendation unit considers the ISF times the Correction Factor as the ISF to determine the recommendation value.

The invention also relates to a method for determining a recommendation value of a control parameter of an insulin infusion device, the method being implemented by the control device as described above and comprising the steps of

- retrieving the user data; and
- determining the recommendation value at least on the basis of the ISF;

wherein the ISF is a function of at least a measured blood glucose level of the plurality of physiological values.

The embodiments, technical effects, and definitions disclosed herein with respect to the control device are also applicable to the method described herein. The method encompasses steps that fully utilize the functionalities and features of the control device described herein. Therefore, all embodiments, technical effects, and definitions pertaining to the device, including but not limited to the evolution of the ISF over time and how it is calculated, are equally applicable to the method. This ensures a comprehensive and unified understanding of both the control device and method aspects of the invention, facilitating the implementation and utilization of the disclosed technology across a range of applications.

The invention also relates to a computer program comprising instructions to cause the control device described above to execute the steps of the method described above.

**Brief description of the drawings**

Embodiments of the invention will be described below with reference to the drawings, described briefly below:

[Fig.1] shows a method for validating a control algorithm according to one embodiment of the invention; and
[Fig.2] shows a control device according to one embodiment of the invention.

Detailed description of the invention

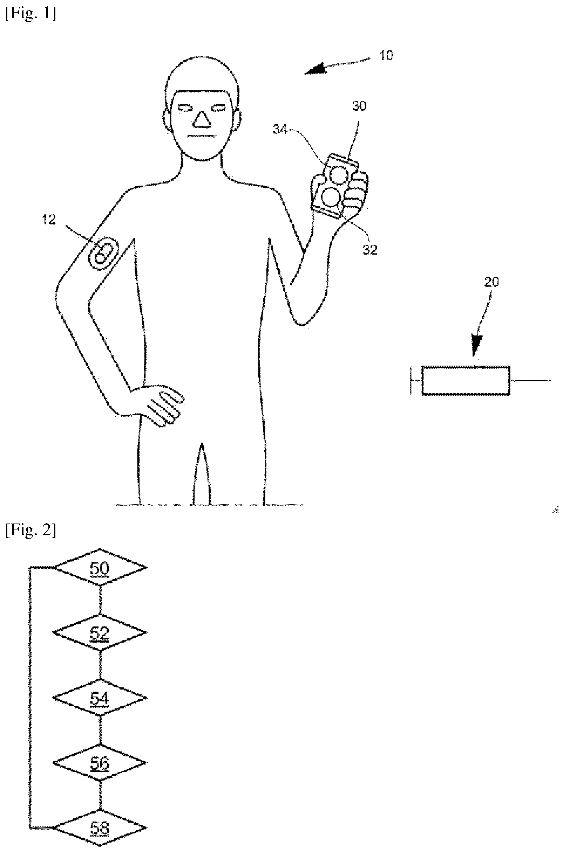

[Fig.1] represents a control device 30 for determining a recommendation value of a control parameter of an insulin infusion device 20. The recommendation value is a recommendation value corresponding to an amount of insulin to be injected at a future time step. The future time step is no more than a few seconds after the nearest timestamp of the physiological values of the unique user. This delay of no more than a few seconds corresponds to the computational time required to determine the recommendation value. The recommendation value can be of any type such as a bolus recommendation or a basal recommendation for example. The terms injected or injection must be understood as a virtual injection in case the training of a reinforcement learning algorithm is made using a simulation wherein the unique user is a virtual user. Such a simulation could be useful to validate or improve an algorithm and therefore reduce the risk for the unique user. Otherwise, the terms injected or injection must be understood as a regular injection.

The control device 30 comprises a retrieving unit 32, the retrieving unit 32 being configured to retrieve user data. Each data of the user data has a timestamp, and the user data is related to a unique user. The user data comprise an amount of insulin infused to the unique user, an amount of carbohydrates ingested by the unique user and a plurality of physiological values of the unique user, the plurality of physiological values of the unique user comprising at least measured blood glucose levels.

A measured blood glucose level is a blood glucose level measured on the unique user. The blood glucose level can be measured by any means such as a Continuous Glucose Monitor (CGM) 12 or a Blood Glucose Monitor (BGM) for example. Preferably, the blood glucose level is measured using a CGM 12. An amount of carbohydrates ingested by the unique user corresponds to an amount of sugar ingested in a meal for example.

The control device 30 also comprises a recommendation unit 34. The recommendation unit 34 is configured to determine the recommendation value at least on the basis of an Insulin Sensitivity Factor (ISF). The ISF is representative of the effect of a determined dose of insulin on a blood glucose level of the unique user. To be more precise, the ISF is representative of the effect of a unit of insulin on the measured blood glucose level of the unique user. A unit of insulin corresponds to the "biological equivalent" of 34.7 $\mu$g pure crystalline insulin.

According to an embodiment, the recommendation unit 34 is configured to determine the recommendation value at least on the basis of a predicted glucose level determined by computing a physiological model of glucose-insulin system representative of the unique user using at least a portion of the user data and the ISF. Such a configuration allows the recommendation unit 34 to accurately determine a recommendation value and therefore helps manage the blood glucose levels of the unique user. Indeed, using an ISF allows the control device 30 to adapt the recommendation value specifically to the unique user. Furthermore, an ISF being a function of at least a measured blood glucose level of the plurality of physiological values allows the physiological model to closely reproduce the physiology of the unique user and therefore allows the recommendation unit to accurately determine a recommendation value. Such a configuration also allows one to more accurately test or fine tune an existing algorithm using a simulator for example. Indeed, an improved physiological model more closely reproduces the physiology of the unique user and therefore allows one to more accurately evaluate the existing algorithm. The physiological model can be of any type such as the Hovorka model, the Bergman minimal model or the Dalla Man Model for example. Preferably, the physiological model is the Hovorka model or a model derived from the Hovorka model.

According to an embodiment, the recommendation unit 34 may also be configured to determine the recommendation value at least on the basis of a Product Integral Derivative (PID) approach and an ISF. The PID is based on at least a measured or predicted blood glucose level and at least a blood glucose level target, the ISF is then used as a weighting factor.

According to an embodiment, the recommendation unit 34 may also be configured to determine the recommendation value at least on the basis of a Neural Network and an ISF. The Neural Network uses as an input at least an amount of insulin infused to the unique user, at least an amount of carbohydrates ingested by the unique user and at least a physiological value of the unique user and outputs a raw recommendation value, the ISF is then used as a weighting factor. The Neural Network could be of any type and trained as follows:

- Collect Data;
- Preprocess Data;
- Design a neural network architecture such as a regression model for example;
- Define the input features;
- Define the output features;
- Train the neural network using the training preprocessed data;
- Validate the model's performance; and
- Evaluate the model's performance using metrics such as Mean Absolute Error (MAE) or Mean Squared Error (MSE) for example.

The recommendation unit 34 is configured to determine the recommendation value at least on the basis of a predicted glucose level determined by computing a physiological model of glucose-insulin system representative of the unique user using an action speed of insulin wherein the action speed of insulin is an increasing function of at least a measured blood glucose level of the plurality of physiological values. Such a configuration allows one to obtain a better Insulin On Board (IOB) value and therefore allows the recommendation unit 34 to more accurately determine a recommendation value. The at least a measured blood glucose level of the plurality of physiological values corresponds to the last measured blood glucose level of the plurality of physiological values or the few last measured blood glucose levels of the plurality of physiological values, or an average of the few last measured blood glucose levels of the plurality of physiological values.

It must be understood that, in the present invention, the ISF is not a function of a Total Daily Dose nor, *a fortiori,* a mean of several past Total Daily Doses. The Total Daily Dose being the total amount of insulin infused to the unique user over the course of a day. Indeed, the ISF is not a constant of glycemia level.

The ISF is a function of at least a measured blood glucose level of the plurality of physiological values. Using an ISF being a function of at least a measured blood glucose level allows the recommendation unit 34 to more accurately determine a recommendation value fitting the unique user's needs as the ISF can vary from one unique user to another and from one measured blood glucose level to another. The ISF being a function of at least a measured blood glucose level of the plurality of physiological values imply that the ISF changes depending on the measured blood glucose level and therefore changes over time if the measured blood glucose level is not constant over time. The ISF is a function of measured blood glucose levels having a timestamp not older than one to three hours prior to the present time and preferably two hours prior to the present time. Such a configuration allows the recommendation unit 34 to more accurately determine the recommendation value as measured blood glucose levels having older timestamps are not as representative of the unique user state as the measured blood glucose levels having younger timestamps.

The ISF is a decreasing function of at least a measured blood glucose level of the plurality of physiological values wherein the considered at least a measured blood glucose level of the plurality of physiological values is greater than 100 mg/dl. Such a configuration allows the control device 30 to accurately determine a recommendation value and therefore helps manage the blood glucose levels of the unique user. Indeed, an ISF being a decreasing function of at least a measured blood glucose level of the plurality of physiological values allows the recommendation 34 unit to accurately determine a recommendation value.

The ISF can be computed as a function of at least a measured blood glucose level of the plurality of physiological values as follows:

$$\text{ISF} = m \times \text{Gly} + b$$

Gly being the considered at least a measured blood glucose level of the plurality of physiological values such as Gly being the closest, in time, measured blood glucose level for example;

m being a factor; and
b being a constant.

Such a configuration allows the control device 30 to accurately determine a recommendation value and therefore helps manage the blood glucose levels of the unique user. Indeed, an ISF being a decreasing function of at least a measured blood glucose level of the plurality of physiological values allows the recommendation unit 34 to accurately determine a recommendation value.

According to an embodiment, the ISF is a piecewise affine function of Gly with m=m1 and b=b1 for Gly below 100 mg/dl, m=m2 and b=b2 for Gly in [100; 160 [ mg/ dl and m=m3 and b=b3 for Gly above 160 mg/dl. Such a configuration allows the ISF to be a closer representation of a physiological state of the unique user. Therefore, b varies depending on Gly such as b1 in [-50; -30] for Gly below 100 mg/dl, b2 in [150; 170] for Gly in [100; 160[ mg/dl and b3 in [50; 70] for Gly above 160 mg/dl. Such a configuration allows the ISF to be a closer representation of a physiological state of the unique user. According to an embodiment, b varies depending on Gly by smoothly transitioning from -50 to 170 according to the previously described evolutions of b. According to another embodiment, ISF is a piecewise affine function with an undefined number of pieces. Furthermore, m varies depending on Gly such as m1 in [1; 1.5] for Gly below 100 mg/dl, m2 in [-0.8; -0.6] for Gly in [100; 160[ mg/ dl and m3 in [-0.2; 0] for Gly above 160 mg/dl. Such a configuration allows the ISF to be a closer representation of a physiological state of the unique user. According to an embodiment, m varies depending on Gly by smoothly transitioning from 1 to -0.8 according to the previously described evolutions of m.

According to another embodiment, ISF is a piecewise affine function with an undefined number of pieces. The functions in the piecewise affine function are non linear exponential functions for instance ISF(Gly) = a $\times$ e^$((c \times$ Gly$))$.

According to an embodiment, functions in the piecewise affine function are logarithmic functions a $\times$ log(Gly)+ b.

Note that previously described examples of piecewise affine functions are linear and nonlinear functions. The piecewise affine functions could be a mix of linear and nonlinear functions such as linear for Gly < 100 mg/dL and nonlinear for Gly >=100 mg/dL for example.

The control device 30 is configured to modify m and b as well as m1, m2, m3, b1, b2 and b3 over time. Such a configuration allows the control device 30 to more precisely adapt to the specificities of the unique user. m and b as well as m1, m2, m3, b1, b2 and b3 can be modified over time using any known method such as the autolearning method described in details above applied to the ISF for example.

The ISF preferably depends on at least one past ISF. Such a configuration allows the recommendation unit 34 to accurately determine a recommendation value and therefore helps manage the blood glucose levels of the unique user while reducing the risk for the unique user. Indeed, an ISF depending on at least one past ISF allows one to avoid abrupt changes in the determination of the recommendation value. A past ISF is an ISF previously calculated/computed. Such a past ISF can be, for example, calculated the same way as the ISF or can be a past ISF initialized arbitrarily by a healthcare professional for example.

Following this reasoning, the ISF depends on an average of several past ISF. Such a configuration allows the recommendation unit 34 to further smooth the changes in the determination of the recommendation value and therefore reduce the risk for the unique user.

According to an embodiment, the ISF depends on an autolearned glycemia factor such as ISF = avgISF * Gly * glyF
wherein:

avgISF being the average of all the computed ISF over the course of a week; and
glyF being a glycemia factor.

According to an embodiment, the ISF depends on an autolearned glycemia factor such as ISF = ALISF * Gly * glyF
wherein:
ALISF being the autolearned ISF;

Such a configuration allows the recommendation unit 34 to accurately determine a recommendation value and therefore helps manage the blood glucose levels of the unique user by adapting to the unique user over time. Such a configuration is especially useful when the variability of the unique user's ISF depending on the measured blood glucose level is high. Such a configuration also allows the recommendation unit 34 to accurately determine a recommendation value and therefore helps manage the blood glucose levels of the unique user by adapting to the unique user over time. Indeed, the ISF of the unique user can vary depending on several factors such as illness unrelated to diabetes for example. According to the present invention, the term diabetes refers to dependence on external insulin.

The term "autolearned ISF" relates to an ISF determined using an autolearning method. The autolearning method could be of any type such as a process through which a system, typically a computer program or an artificial intelligence, autonomously acquires knowledge, skills, or behaviors without explicit programming or direct human intervention. Autolearning systems often rely on algorithms, data, and feedback mechanisms to improve their performance over time, adapting to changing conditions or tasks through self-adjustment and refinement.

According to an embodiment, the autolearned ISF is determined by creating a plurality of time segments, each time segment having an ISF, in order to group user data at least based on their timestamp; creating a segment correction value for each time segment, the segment correction values being based at least on one past ISF and on at least one physiological value of the plurality of physiological values during a time segment of the plurality of time segments; creating the autolearned ISF for a future determined period of time, wherein the autolearned ISF is based at least on a segment correction value of the segment correction values and the ISF of the determined period of time. Using segments allows one to accurately determine the autolearned ISF as each segment correction values can be created differently, time segment by time segment or depending on a specific interest of a time segment for example.

The ISF may also depends on an autolearned glycemia factor as ISF = pISF * Gly * ALglyF wherein:

> pISF being a past ISF;
> avgISF being an average of past ISF such as the closest, in time, past five ISF for example;
> ALglyF being the autolearned glycemia factor.

Such a configuration allows the recommendation unit to accurately determine a recommendation value and therefore helps manage the blood glucose levels of the unique user by adapting to the unique user over time. Such a configuration is especially useful when the variability of the unique user's ISF depending on the measured blood glucose level is high. The pISF can be replaced by avgISF. Indeed, ISF = avgISF * Gly * ALglyF allows one to further smooth the changes in the determination of the recommendation value and therefore reduce the risk for the unique user.

According to the present invention, the term "autolearned glycemia factor" relates to a glycemia factor determined using an autolearning method. The autolearning method could be of any type such as a process through which a system, typically a computer program or an artificial intelligence, autonomously acquires knowledge, skills, or behaviors without explicit programming or direct human intervention. Autolearning systems often rely on algorithms, data, and feedback mechanisms to improve their performance over time, adapting to changing conditions or tasks through self-adjustment and refinement.

According to an embodiment, the autolearned glycemia factor is determined by creating a plurality of time segments, each time segment having a glycemia factor, in order to group user data at least based on their timestamp; creating a segment correction value for each time segment, the segment correction values is based at least one past glycemia factor and at least one physiological value of the plurality of physiological values during a time segment of the plurality of time segments; creating the autolearned glycemia factor for a future determined period of time, wherein the autolearned glycemia factor is based at least on a segment correction value of the segment correction values and the glycemia factor of the determined period of time. Using segments allows one to accurately determine autolearned glycemia factor as each segment correction values can be created differently, time segment by time segment or depending on a specific interest of a time segment for example.

According to an embodiment, the ISF is a combination of previously described embodiment as the ISF is a decreasing function of at least a measured blood glucose level of the plurality of physiological values and depends on an autolearned ISF and an autolearned glycemia factor. The ISF depends on an autolearned ISF and an autolearned glycemia factor such as ISF = ALISF * Gly * ALglyF. Such a configuration allows one to accurately determine a recommendation value and therefore helps manage the blood glucose levels of the unique user by adapting to the unique user over time. Indeed, both the ISF and the glyF of the unique user can vary depending on several factors such as illness unrelated to diabetes for example.

The recommendation unit 34 is configured to determine the recommendation value at least on the basis of the ISF and a Correction Factor. The Correction Factor is a factor applied to the ISF and allows the recommendation unit 34 to take into account particular situations of the unique user such as illness unrelated to diabetes for example. The recommendation unit 34 considers the ISF times the Correction Factor as the ISF to determine the recommendation value.

The control device 30 also comprises an insulin delivery unit 20. The preferred insulin delivery unit 20 is a subcutaneous insulin delivery device configured to deliver exogenous insulin in a subcutaneous tissue of the patient in response to an insulin delivery control signal such as an insulin pump. In particular continuously infused insulin and/or bolus insulin.

According to an embodiment, the control device 30 comprises a converter unit not represented in the drawings. The converter unit is configured to convert the recommended value of insulin into a control parameter of the fluid infusion device 20. The control parameter takes the form of a bolus and/or a basal. Such a configuration allows the infusion device to infuse the recommendation value to the unique user.

As shown in [Fig.2], the invention also relates to a method for determining a recommendation value of a control parameter of an insulin infusion device 20 as described above. The embodiments, technical effects, and definitions disclosed herein with respect to the control device 30 are also applicable to the method described herein. The method encompasses steps that fully utilize the functionalities and features of the control device 30 described herein. Therefore, all embodiments, technical effects, and definitions pertaining to the device, including but not limited to the evolution of the ISF over time and how it is calculated, are equally applicable to the method. This ensures a comprehensive and unified understanding of both the control device 30 and method aspects of the invention, facilitating the implementation and utilization of the disclosed technology across a range of applications.

The method comprises the steps of:

- retrieving 50 the user data; and
- determining 58 the recommendation value at least on the basis of the ISF;

wherein the ISF is a function of at least a measured blood glucose level of the plurality of physiological values.

According to an embodiment wherein the SF depends on the autolearned glycemia factor such as ISF = ALISF * Gly * glyF, the method also comprises the steps of:

- creating a plurality of time segments 52, each time segment having an ISF, in order to group user data at least based on their timestamp;
- creating a segment correction value for each time segment 54, the segment correction values being based at least on one past ISF and on at least one physiological value of the plurality of physiological values during a time segment of the plurality of time segments;
- creating the autolearned ISF for a future determined period of time 56, wherein the autolearned ISF is based at least on a segment correction value of the segment correction values and the ISF of the determined period of time.

Using segments allows one to accurately determine the autolearned ISF as each segment correction values can be created differently, time segment by time segment or depending on a specific interest of a time segment for example.

The invention also relates to a computer program comprising instructions to cause the control device 30 described above to execute the steps of the method described above.

The invention also relates to a control system 10 comprising an insulin infusion device 20, a CGM 12 and control device 30 comprising a recommendation unit 34 and a retrieving unit 32.

While exemplary embodiments of the invention have been described, it will be understood by those skilled in the art that various changes, omissions and/or additions may be made, and equivalents may be substituted for elements thereof without departing from the spirit and scope of the invention. In addition, many modifications might be made to adapt a particular situation or material to the teachings of the invention without departing from the scope thereof. Therefore, it is intended that the invention is not limited to the particular embodiments disclosed for carrying out this invention, but that the invention will include all embodiments falling within the scope of the appended claims. Moreover, unless specifically stated, any use of the terms first, second, etc. do not denote any order or importance, but rather the terms first, second, etc. are used to distinguish one element from another.

**Claims**

1. A control device (30) for determining a recommendation value of a control parameter of an insulin infusion device (20), the control device (30) comprising:

    • a retrieving unit (32), the retrieving unit (32) being configur ed to retrieve user data, each data of the user data having a t timestamp and the user data being related to a unique user, t he user data comprising at least:

        • an amount of insulin infused to the unique user;
        • an amount of carbohydrates ingested by the uniqu e user;
        • a plurality of physiological values of the unique us er, the plurality of physiological values of the uniq ue user comprising at least measured blood glucos e levels;

    • a recommendation unit (34), the recommendation unit (34) being configured to determine the recommendation value at least on the basis of an Insulin Sensitivity Factor (ISF);

    wherein, the ISF is a function of at least a measured blood glucose level of the plurality of physiological values.

2. A control device (30) for determining a recommendation value of a control parameter of an insulin infusion device (20) according to claim 1, wherein the ISF is a decreasing function of at least a measured blood glucose level of the plurality of physiological values.

3. A control device (30) for determining a recommendation value of a control parameter of an insulin infusion device (20) according to claim 1, wherein the ISF is a function of at least a measured blood glucose level of the plurality of physiological values as follows:

$$ISF = m \times Gly + b$$

Gly being the considered at least a measured blood glucose level of the plurality of physiological values such as Gly being the closest, in time, measured blood glucose level for example; m being a factor; and b being a constant.

4. A control device (30) for determining a recommendation value of a control parameter of an insulin infusion device (20) according to any of the claims 1 to 3, wherein the recommendation unit (34) is configured to determine the recommendation value at least on the basis of a predicted glucose level determined by computing a physiological model of glucose-insulin system representative of the unique user using at least a portion of the user data and the ISF.

5. A control device (30) for determining a recommendation value of a control parameter of an insulin infusion device (20) according to claim 4, wherein the recommendation unit (34) is configured to determine the recommendation value at least on the basis of a predicted glucose level determined by computing a physiological model of glucose-insulin system representative of the unique user using an action speed of insulin wherein the action speed of insulin is an increasing function of at least a measured blood glucose level of the plurality of physiological values.

6. A control device (30) for determining a recommendation value of a control parameter of an insulin infusion device (20) according to any of the claims 1 to 5, wherein the ISF is a decreasing function of at least a measured blood glucose level of the plurality of physiological values and depends on at least one past ISF.

7. A control device (30) for determining a recommendation value of a control parameter of an insulin infusion device (20) according to any of the claims 1 to 6, wherein the ISF is a decreasing function of at least a measured blood glucose level of the plurality of physiological values and depends on an average of several past ISF.

8. A control device (30) for determining a recommendation value of a control parameter of an insulin infusion device (20) according to any of the claims 1 to 7, wherein the ISF is a decreasing function of at least a measured blood glucose level of the plurality of physiological values and depends on an autolearned ISF.

9. A control device (30) for determining a recommendation value of a control parameter of an insulin infusion device (20) according to any of the claims 1 to 8, wherein the ISF depends on an autolearned glycemia factor.

10. A control device (30) for determining a recommendation value of a control parameter of an insulin infusion device (20) according to any of the claims 1 to 9, wherein the ISF is a decreasing function of at least a measured blood glucose level of the plurality of physiological values and depends on an autolearned ISF and an autolearned glycemia factor.

11. A control device (30) for determining a recommendation value of a control parameter of an insulin infusion device (20) according to any of the claims 1 to 10, wherein the recommendation unit (34) is configured to determine the recommendation value at least on the basis of the ISF and a Correction Factor.

12. A method for determining a recommendation value of a control parameter of an insulin infusion device (20), the method being implemented by the control device (30) of claim 1 and comprising the steps of:

    • retrieving (50) the user data; and
    • determining (52) the recommendation value at least on the basis of the ISF;

    wherein the ISF is a function of at least a measured blood glucose level of the plurality of physiological values.

13. A computer program comprising instructions to cause the control device (20) of claim 1 to execute the steps of the method of claim 12.

[Fig. 1]

[Fig. 2]

Europäisches
Patentamt
European
Patent Office
Office européen
des brevets

# EUROPEAN SEARCH REPORT

**Application Number**

EP 25 17 4485

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/269585 A1 (GINSBERG BARRY H [US]) 30 October 2008 (2008-10-30) | 1-4,6-13 | INV. G16H20/17 |
| Y | * paragraph [0012] - paragraph [0020] * * paragraph [0032] - paragraph [0035]; figure 2 * * paragraph [0036] - paragraph [0050]; figure 3 * * claims 1-12 * | 5 | G16H50/50 |
| X | US 2019/228853 A1 (BENGTSSON HENRIK [DK] ET AL) 25 July 2019 (2019-07-25) | 1-4,6-13 | |
| Y | * paragraph [0026] - paragraph [0037] * | 5 | |
| Y | US 2016/038673 A1 (MORALES CARLOS [US]) 11 February 2016 (2016-02-11) * paragraph [0042] - paragraph [0119]; figures 5-8 * | 5 | |
| A | TRISTAN BEOLET ET AL: "End-to-end Offline Reinforcement Learning for Glycemia Control", ARXIV.ORG, CORNELL UNIVERSITY LIBRARY, 201 OLIN LIBRARY CORNELL UNIVERSITY ITHACA, NY 14853, 16 October 2023 (2023-10-16), XP091637261, * the whole document * | 1-13 | |

**TECHNICAL FIELDS SEARCHED (IPC)**

G16H

The present search report has been drawn up for all claims

| Place of search | Date of completion of the search | Examiner |
|---|---|---|
| Munich | 23 September 2025 | Eichenauer, Lars |

EPO FORM 1503 03.82 (P04C01)

EP 4 651 145 A1

## ANNEX TO THE EUROPEAN SEARCH REPORT
## ON EUROPEAN PATENT APPLICATION NO.

EP 25 17 4485

23-09-2025

| Patent document cited in search report | | | Publication date | Patent family member(s) | | | Publication date |
|---|---|---|---|---|---|---|---|
| US 2008269585 | A1 | | 30-10-2008 | CA | 2498682 | A1 | 01-09-2005 |
| | | | | EP | 1571582 | A2 | 07-09-2005 |
| | | | | ES | 2732076 | T3 | 20-11-2019 |
| | | | | JP | 4514141 | B2 | 28-07-2010 |
| | | | | JP | 2005315855 | A | 10-11-2005 |
| | | | | US | 2005192494 | A1 | 01-09-2005 |
| | | | | US | 2008269585 | A1 | 30-10-2008 |
| US 2019228853 | A1 | | 25-07-2019 | CN | 109661706 | A | 19-04-2019 |
| | | | | CN | 109690685 | A | 26-04-2019 |
| | | | | EP | 3482314 | A1 | 15-05-2019 |
| | | | | EP | 3482319 | A1 | 15-05-2019 |
| | | | | JP | 7042791 | B2 | 28-03-2022 |
| | | | | JP | 7217698 | B2 | 03-02-2023 |
| | | | | JP | 2019530914 | A | 24-10-2019 |
| | | | | JP | 2019530915 | A | 24-10-2019 |
| | | | | JP | 2022043110 | A | 15-03-2022 |
| | | | | JP | 2022043112 | A | 15-03-2022 |
| | | | | MA | 45579 | A | 15-05-2019 |
| | | | | MA | 45601 | A | 15-05-2019 |
| | | | | US | 2019228853 | A1 | 25-07-2019 |
| | | | | US | 2019348164 | A1 | 14-11-2019 |
| | | | | WO | 2018007160 | A1 | 11-01-2018 |
| | | | | WO | 2018007161 | A1 | 11-01-2018 |
| US 2016038673 | A1 | | 11-02-2016 | EP | 2973095 | A2 | 20-01-2016 |
| | | | | ES | 2671001 | T3 | 04-06-2018 |
| | | | | HK | 1218580 | A1 | 24-02-2017 |
| | | | | US | 2016038673 | A1 | 11-02-2016 |
| | | | | WO | 2014149536 | A2 | 25-09-2014 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090054753 A1